# FASCICULE DE BREVET EUROPEEN

(11) **EP 1 610 691 B1**
(45) Date de publication et mention de la délivrance du brevet: **21.08.2013**
(21) Numéro de dépôt: 04722308.6
(22) Date de dépôt: 22.03.2004
(51) Int. Cl.: A61B 17/00, A61B 17/34

(54) **DISPOSITIF POUR LA MISE EN PLACE D UN IMPLANT VASCULAIRE**
VORRICHTUNG ZUM PLATZIEREN EINES GEFÄSSIMPLANTATS
DEVICE FOR PLACING A VASCULAR IMPLANT

(30) Priorité: 10.04.2003 FR 0350096
(43) Date de publication de la demande: 04.01.2006
(73) Titulaire: Mialhe, Claude, 83300 Draguignan (FR)
(72) Inventeur: Mialhe, Claude, 83300 Draguignan (FR)
(74) Mandataire: Decobert, Jean-Pascal
(86) Numéro de dépôt international: PCT/FR2004/050118
(87) Numéro de publication internationale: WO 2004/091410

(56) Documents cités:
- WO-A-02/19926
- US-A- 5 320 639
- US-A- 6 030 364
- US-A1- 2002 042 622
- US-A1- 2002 107 482
- US-B1- 6 391 036

## Description

La présente invention concerne un dispositif de mise en place d'un implant.

Elle trouvera son application en particulier pour l'utilisation de cathéters ou de tout autre instrument permettant la mise en place d'un implant dans un vaisseau du corps humain ou animal.

L'invention pourra être utilisée pour la mise en place d'objets dans un vaisseau sous forme d' implants du type stents ou encore d'un dispositif d'occlusion vasculaire tel que présenté, par exemple, dans le document WO-A 02 19 926, ou encore des implants de type « coil ».

L'invention pourra également s'appliquer à la mise en place de dispositifs d'occlusion trans-pariétale pour obturer une ouverture dans le vaisseau. Le terme implant s'entend donc ici au sens large.

La mise en place d'implants dans un vaisseau nécessite de former une ouverture trans-pariétale (par pénétration des différentes couches tissulaires) pour parvenir à la lumière interne dudit vaisseau.

D'une façon générale, on utilise à cet effet un instrument de dilatation comportant une partie d'extrémité effilée apte à réaliser une augmentation progressive du diamètre du passage réalisé dans la paroi vasculaire.

D'une façon générale, on commence par introduire une aiguille au travers de la paroi vasculaire et on met en place un élément de guidage généralement sous forme d'un câble de guidage dont l'extrémité est maintenue en position dans la lumière. Le câble de guidage permet d'enfiler d'autres instruments et de les guider au travers de l'ouverture réalisée dans la paroi vasculaire. Ces instruments comprennent généralement un élément introducteur avec une partie d'extrémité effilée centrale permettant l'augmentation progressive du diamètre de l'ouverture pariétale. Cet élément effilé central est entouré d'une gaine externe qui vient finalement à être introduit au travers de la paroi vasculaire après que la partie effilée centrale ait produit son effet. Cette dernière peut alors être retirée alors que la gaine externe est maintenue en position et utilisée pour pratiquer le geste chirurgical souhaité.

Par exemple, c'est par la gaine externe résiduelle qu'il est possible d'opérer la mise en place d'un implant vasculaire. Durant ces opérations, le câble de guidage peut rester en position.

La technique usuelle précédemment décrite présente de multiples inconvénients.

En premier lieu, la partie effilée d'introduction progressive est réalisée dans un élément interne à l'instrument jusqu'à parvenir au diamètre de la gaine externe. Dans ce cadre, la partie effilée interne encombre le volume intérieur de la gaine externe pendant toute une partie de l'opération, ce qui exclut notamment toute possibilité de présence d'un autre élément fonctionnel ou implantable (par exemple un implant vasculaire) dans le volume intérieur de la gaine externe, et ce avant d'avoir finalisé l'introduction.

Un autre inconvénient des introducteurs actuels est qu'il subsiste une zone de transition de dimension entre l'élément effilé interne et la paroi extérieure de la gaine externe : cela crée une discontinuité de diamètre, ce qui peut être dommageable à la continuité du mouvement d'introduction et abîmer la paroi interne des vaisseaux.

Le document WO-A- 02 19 926 précité concerne un dispositif d'occlusion vasculaire comportant deux organes expansibles pour sa fixation par appui sur deux portions de la paroi du vaisseau. Il comprend en outre une partie intermédiaire déformable en torsion à un degré ajustable selon la position relative des deux organes expansibles de façon à créer une zone de striction maximale définissant un degré d'occlusion. Ce document présente également un procédé d'utilisation ainsi qu'un appareil de mise en place de ce dispositif d'occlusion.

Les instruments de dilatation actuellement connus s'avèrent peu adaptés à des techniques du type décrit dans WO-A-02 19 926 notamment parce qu'ils excluent la présence d'un élément fonctionnel dans leur volume intérieur avant la fin de l'introduction dans le vaisseau.

On connaît du document US-A1-2002 0042622 un dispositif médical pour des interventions du type anastomose. Ce dispositif comporte un trocart servant à transfixier la paroi d'un vaisseau. Ce trocart à l'extrémité effilée et ouvrable a cependant une fonction fort limitée suivant cette antériorité car il ne sert qu'à la perforation.

On connaît du document US - A- 5,320,639 un dispositif de délivrance d'un bouchon d'occlusion vasculaire. Le dispositif comprend un canal dans lequel s'insère le bouchon qui est mis en place au contact d'une ouverture latérale de la paroi d'un vaisseau. Le canal peut avoir une extrémité effilée et ouvrable. Ce dispositif est destiné à une utilisation spécifique d'occlusion par l'extérieur d'un vaisseau vasculaire.

La présente invention permet de remédier aux inconvénients des dispositifs connus jusqu'à présent. Ainsi, elle permet une introduction progressive, une augmentation du diamètre de l'ouverture pariétale et un centrage dans un vaisseau par un seul organe externe qui participe aussi à la libération de l'implant.

La présente invention ne nécessite pas l'encombrement du volume intérieur du dispositif car il n'y a pas de partie centrale effilée à retirer.

Le volume intérieur du dispositif est donc maintenu libre, pour l'intégration notamment, dès le départ, d'un implant dans le dispositif. Il est ainsi possible, par exemple, de proposer à la vente un instrument intégralement équipé de l'implant à mettre en place.

Il s'avère de surcroît possible d'effectuer plusieurs opérations dans le vaisseau au cours d'un même geste chirurgical : en formant un système à mémoire de forme, l'invention peut assurer une première introduction, être refermée pour atteindre un autre secteur vasculaire et y être réemployée.

L'invention comporte de façon caractéristique une enveloppe avec un dispositif de dilatation réalisant à la fois une fonction de dilatation et une fonction de contention de l'implant directement appliqué au moins partiellement sur sa paroi interne. On réalise ainsi un ensemble compact (moins de gaines superposées) facilitant la mise en place tout en étant guidable par un guide central conventionnel du type câble de guidage.

L'enveloppe sert donc aussi d'organe de centrage à l'intérieur du vaisseau. Un volume libre est par ailleurs préservé au centre de l'ensemble, par exemple pour le passage d'un ballon d'expansion.

On notera en outre que l'implant est intégralement protégé lors des mouvements de mise en place. Lors de la libération d'un organe expansible, le nez effilé de l'enveloppe assure la progressivité de la libération évitant tout effet de « saut » habituellement rencontré par variation brusque du diamètre d'un stent lorsqu'il est expulsé de son logement.

D'autres buts et avantages apparaîtront au cours de la description qui suit d'un mode préféré de réalisation de l'invention qui n'est cependant pas limitatif.

La présente invention concerne un dispositif pour la mise en place d'un implant vasculaire comprenant :
- un dispositif de dilatation d'un vaisseau avec une enveloppe externe et une partie d'extrémité effilée pour l'introduction dans le vaisseau, ladite partie d'extrémité étant constituée d'un nez formé à l'extrémité distale de l'enveloppe externe et le dispositif de dilatation, comportant des moyens d'ouverture du nez avec au moins deux fentes longitudinales partageant le nez en plusieurs segments déployables pour ouvrir le nez
- un implant placé dans l'enveloppe externe
caractérisé par le fait
- que l'implant comprend un organe expansible s'appliquant sur la paroi interne de l'enveloppe externe
- qu'il présente des moyens de translation de l'implant relativement à l'enveloppe externe de sorte de faire appuyer l'organe expansible sur la paroi interne du nez pour en déployer les segments

Ce dispositif pourra se présenter dans des variantes avantageuses mais non limitatives indiquées ci-après :
- les moyens de translation comportent une gaine interne montée coulissante dans l'enveloppe externe et poussant l'organe expansible
- l'implant comporte un deuxième organe expansible creux et une partie intermédiaire creuse et déformable en torsion
- le deuxième organe expansible s'applique sur la paroi interne de la gaine interne
- la gaine interne est montée coulissante et en rotation dans l'enveloppe externe
- Il comporte une poignée de préhension solidaire de l'enveloppe externe
- il comporte une poignée de préhension solidaire de la gaine interne
- la poignée de préhension de la gaine interne est située en arrière de la poignée de préhension de l'enveloppe externe et qu'elle comporte une entretoise amovible intercalée entres lesdites poignées pour maintenir leur écartement.
- les segments déployables sont joints ponctuellement le long des fentes en position de fermeture du nez
- il comporte une jonction ponctuelle par fente entre les segments
- le nez comporte un passage central résiduel
- le nez présente une mémoire de forme de sorte à être fermé par défaut lorsque les moyens d'ouverture sont inactifs.
- il comporte un pousseur monté coulissant dans la gaine interne et apte à s'appliquer sur l'extrémité libre du deuxième organe expansible
- il comporte une poignée de préhension solidaire du pousseur située en arrière d'une poignée de préhension solidaire de la gaine interne et qu'il comporte une entretoise amovible intercalée entre lesdites poignées pour maintenir leur écartement.
- il comporte des moyens de réglage de la position angulaire de la gaine interne
- il comporte un chenal central suivant l'axe de l'enveloppe externe pour le passage d'un câble de guidage.

Les dessins ci-joints sont donnés à titre d'exemples et ne sont pas limitatifs de l'invention. Ils représentent seulement un mode de réalisation de l'invention et permettront de la comprendre aisément.

La figure 1 est une vue générale de côté d'un dispositif de mise en place selon l'invention.

La figure 2 en est une vue en coupe.

La figure 3 illustre un exemple de réalisation d'un câble de guidage.

La figure 5 est une vue de côté d'une enveloppe externe et la figure 4 en est une vue de face.

La figure 6 illustre une configuration possible mais non limitative d'un dispositif implantable pour l'occlusion vasculaire.

Les figures 7 et 8 illustrent respectivement des vues de côté et de face d'une entretoise utilisable dans le dispositif de mise en place de l'invention.

La figure 9 est une vue de côté d'une gaine interne utilisable selon l'invention et la figure 10 est une vue de côté d'un pousseur.

Les figures 11 à 18 illustrent chronologiquement différentes phases d'utilisation du dispositif selon l'invention pour la mise en place d'un implant.

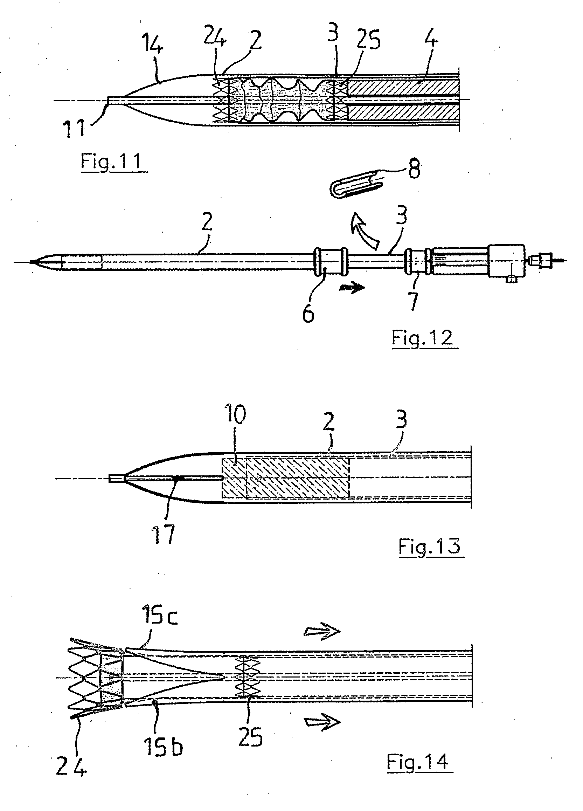

Dans ce cadre, la figure 11 est une vue partielle en coupe du dispositif de dilatation équipé d'un implant.

La figure 12 montre une étape de modification de la configuration de l'instrument avec enlèvement d'une entretoise.

La figure 13 montre une vue de côté partielle de l'invention montrant un exemple de réalisation d'un nez effilé sur l'enveloppe externe.

La figure 14 montre le mouvement relatif de différents organes du dispositif de l'invention pour l'ouverture du nez.

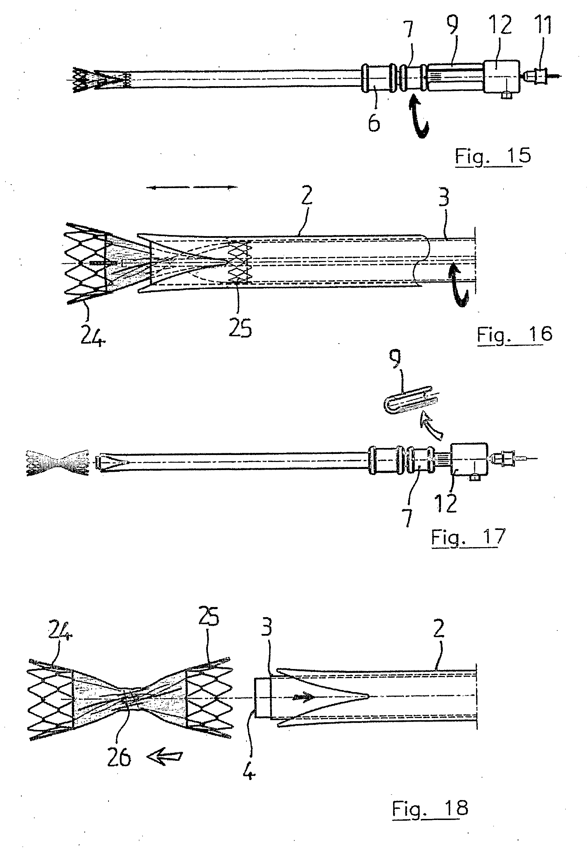

La figure 15 montre une autre phase de fonctionnement du dispositif de mise en place selon l'invention.

La figure 16 en est une vue de détail.

La figure 17 montre une dernière phase d'utilisation de l'instrument avec enlèvement d'une seconde entretoise.

La figure 18 en est une vue de détail.

Le terme de dilatation s'entend, dans le cadre de l'invention, aussi bien :
- de la dilatation d'une zone rétrécie par un instrument plein de taille progressivement croissante. L'extrémité effilée permet de réaliser un élargissement canalaire en forçant l'écartement progressif des parois ou par extension l'écartement des tissus sur la zone de pénétration. Le profil du dilatateur permet aussi l'autocentrage de l'instrument. Ce type de profil limite le risque de lésion de la paroi interne des vaisseaux souvent recouverte de plaques calcifiées,
- utilisation pour la dilatation « thérapeutique » telle que réalisée par inflation d'un ballon avec endoprothèse. La pression exercée sur les parois permet de fracturer les plaques calcifiées et d'espérer une augmentation de calibre persistante après déflation du ballon. Un résultat incomplet est complété par la mise en place d'une endoprothèse.

Le document WO-A 02 19 926 présente un dispositif d'occlusion vasculaire particulier comportant deux organes expansibles d'extrémité et une partie intermédiaire déformable en torsion par modification de la position angulaire relative des deux organes expansibles d'extrémité.

Dans la description qui suit, on décrit l'utilisation du dispositif selon l'invention pour une mise en place du dispositif d'occlusion vasculaire indiqué dans le document précédent. Cela étant, cet exemple d'utilisation est purement indicatif et ne saurait être considéré comme une limitation de l'application de la présente invention. En particulier, le dispositif peut présenter un implant d'autres types, notamment avec un seul organe expansible sur sa largeur. L'organe expansible peut être auto-expansible ou expansible par ballon.

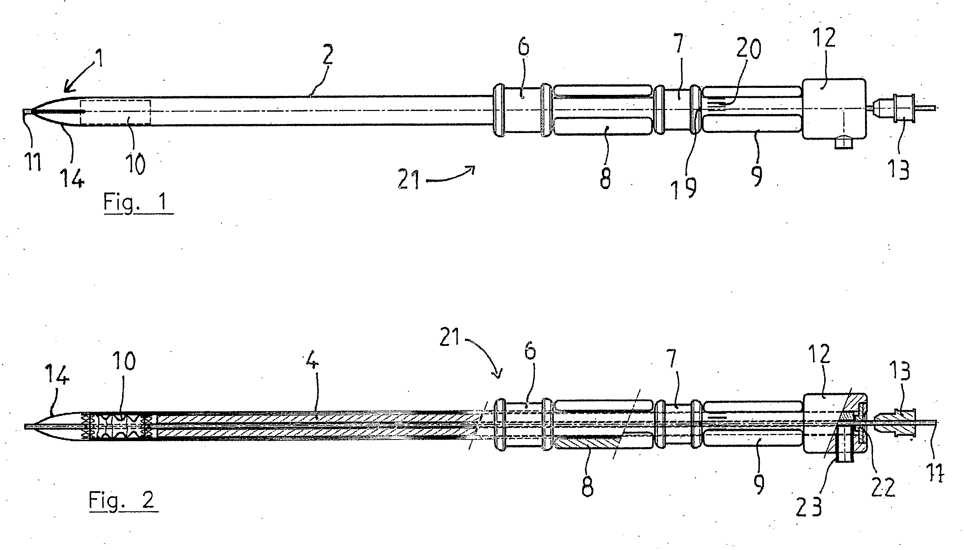

Les figures 1 et 2 présentent en vue de côté et en coupe un dispositif de mise en place selon l'invention. Ce dispositif 21 comporte dans sa partie avant un dispositif de dilatation 1 d'un vaisseau. A l'opposé du dispositif de dilatation 1, le dispositif de mise en place 21 comporte une partie permettant la manipulation par le praticien. En particulier, des poignées 6, 7 et 12 sont constituées pour la préhension. La poignée 12 peut intégrer ou recevoir différents accessoires telle une valve 22 préservant l'étanchéité de l'instrument ainsi qu'un raccord 23 pour la connexion de tuyaux additionnels.

On décrit ci-après plus précisément les différents éléments constitutifs du dispositif de dilatation 1 et du dispositif de mise en place 21 selon l'invention dans le mode de réalisation illustré.

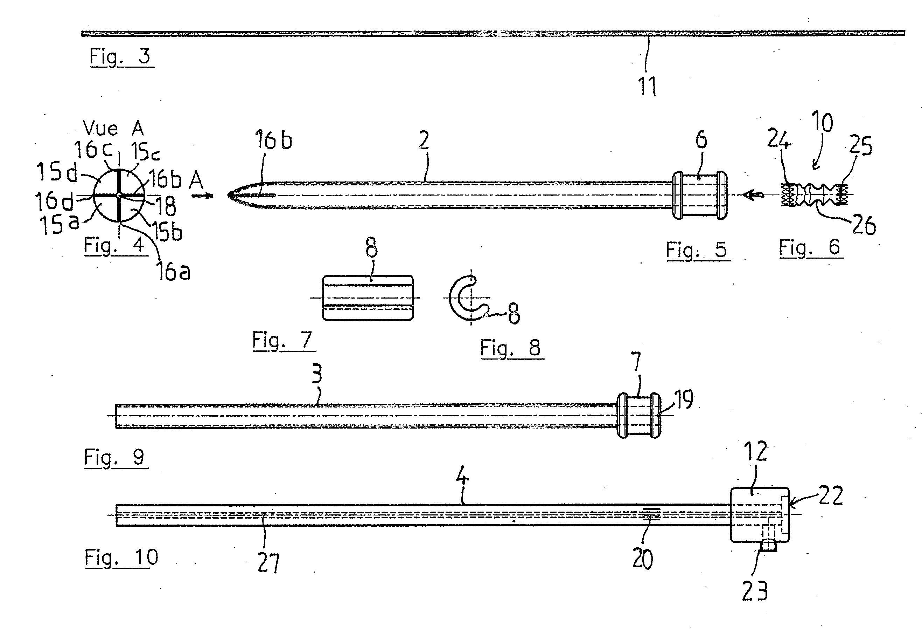

Dans ce cadre, la figure 3 montre la formation d'un câble de guidage 11 (encore appelé fil guide) pouvant être mis en place et préservé dans la partie centrale du dispositif 1 et du dispositif de mise en place 21 durant toutes les phases d'utilisation de l'invention.

Les figures 4 et 5 montrent une enveloppe externe 2 apte à réaliser le corps principal et à délimiter un volume intérieur de travail. L'enveloppe externe comporte une extrémité distale constituée par un nez 14 de forme effilée configurée pour permettre l'introduction dans le vaisseau au travers de sa paroi.

Au cours de l'ufiilisation, le nez 14 est d'abord en position fermée par défaut de façon à constituer le profil d'introduction effilé. Lorsque le nez 14 est suffisamment introduit dans le vaisseau, des moyens d'ouverture sont présents pour permettre d'ouvrir le nez 14.

On présente ci-après un exemple de réalisation de ces moyens d'ouverture. Ainsi, dans le cadre des figures 4 et 5, le nez 14 est équipé d'une pluralité de fentes 16a, 16b, 16c, 16d réalisant une partition du nez 14 en plusieurs segments 15a, 15b, 15c, 15d. Les segments 15a, 15b, 15c, 15d ont ainsi une liberté de mouvement relative permettant d'ouvrir le nez 14 dans un mouvement sensiblement correspondant à celui des pétales d'une fleur.

Le nombre de fentes 16a, 16b, 16c, 16d et leur longueur dans le sens longitudinal du dispositif 1 ne sont pas limités à l'exemple illustré.

Avantageusement, les segments 15a, 15b, 15c, 15d du nez 14 ont une mémoire de forme pour retrouver leur position de repos fermée lorsque les moyens d'ouverture ne sont plus actifs. On peut ainsi ouvrir et femer le nez 14 à plusieurs reprises, selon les besoins du praticien.

On peut prévoir d'autres moyens pour refermer le nez 14, sous forme d'un système de fermeture active par exemple en faufilant un fil dans les différents segments 15a, 15b, 15c, 15d entre l'extrémité du nez 14 et une poignée périphérique : la tension du fil referme le nez 14 et le relâchement peut aussi servir à l'ouverture du nez 14.

A l'extrémité opposée de l'enveloppe externe 2, une poignée 6 est présente pour la préhension par l'opérateur. On notera que l'enveloppe externe 2 délimite un volume intérieur permettant, par exemple, l'introduction d'un dispositif d'occlusion vasculaire 10 présenté à la figure 10 et comportant deux organes expansibles 24, 25 ainsi qu'une partie intermédiaire souple et déformable en torsion 26.

Le dispositif 1 et le dispositif de mise en place 21 comportent en outre une gaine interne 3 apte à être montée coulissante dans le volume interne de l'enveloppe externe 2. Tout comme l'enveloppe externe 2, la gaine interne 3 pourra être constituée par un tronçon sensiblement cylindrique de section circulaire. L'extrémité distale de la gaine interne est laissée libre tandis que l'autre extrémité comporte une poignée 7 permettant la préhension par le praticien.

L'invention comporte également un pousseur 4 visible en figure 10 dans un mode préféré de réalisation dans lequel il possède un chenal central 27 réalisant un passage résiduel dans le coeur du dispositif ainsi qu'une poignée 12 recevant, dans le cas représenté, une valve 22 d'extrémité ainsi qu'un raccord 23 pour des connexions accessoires. Le pousseur 4 est monté à coulissement dans le volume intérieur de la gaine interne 3.

On parvient, par ce montage, à la configuration illustrée aux figures 1 et 2. Les poignées 6, 7 et 12 sont maintenues écartées par le biais d'entretoises 8, 9 constituées de façon amovibles pour pouvoir être successivement ôtées au cours du geste chirurgical.

Le câble de guidage 11 est ici positionné dans le chenal central résiduel 27 du pousseur 4 et son extrémité proximale est associée à un manchon permettant également la manipulation.

Dans l'exemple illustré, l'ouverture du nez 14 s'opère comme suit. Depuis une position fermée, apparaissant par exemple à la figure 11, le praticien enlève la première entretoise 8 de façon à permettre le retrait de l'enveloppe externe 2 relativement à la gaine interne 3 jusqu'à parvenir à la mise en butée des poignées 6, 7. Lors de ce mouvement de retrait, l'extrémité distale de la gaine interne 3 exerce un appui sur la paroi interne de l'enveloppe externe 2 via un organe expansible 24 de l'implant 10. Cet appui provoque le déploiement des segments 15a, 15b, 15c, 15d du nez 14 tel que cela ressort de la figure 14.

Suivant une possibilité, des jonctions ponctuelles 17 sont présentes sur la longueur des fentes 18a, 16b, 16c, 16d de façon à préserver une cohésion réglée entre les différents segments 15a, 15b, 15c, 15d durant la phase d'introduction. Ces jonctions 17 sont néanmoins prévues pour ne pas gêner le déploiement du nez 14 et pour permettre de détacher les différents segments 15a, 15b, 15c, 15d de par l'appui exercé par la gaine interne 3 lors du retrait de l'enveloppe externe 2.

Des soudures ponctuelles pourront faire office des jonctions 17.

On décrit ci-après plus précisément un exemple de réalisation d'un dispositif de mise en place d'implants et ce, dans le cas non limitatif des mises en place d'implants d'occlusion vasculaire tels que représentés en figure 6.

Dans ce cadre, l'implant 10 est positionné dans le volume intérieur de l'enveloppe externe 2 avant le début de l'opération. Plus précisément, l'organe expansible distal 24 est maintenu par la paroi interne de l'enveloppe externe 2. En arrière, l'autre organe expansible 25 est maintenu en position contre la paroi interne de la gaine interne 3. Avantageusement, on met en appui l'extrémité arrière de l'organe expansible 24 sur l'extrémité avant de la gaine interne 3. Parallèlement, l'extrémité arrière de l'organe expansible 25 est mise en appui contre l'extrémité distale du pousseur 4. Cette configuration est bien représentée en figure 11.

Dans un premier temps, le praticien introduit le nez 14 au travers de la paroi vasculaire jusqu'à parvenir à l'endroit d'implantation désiré. A ce stade, l'entretoise 8 est enlevée, ce qui permet au praticien de retirer l'enveloppe externe 2 relativement à la gaine interne 3 et ce par le biais de la poignée 6. Ce mouvement est illustré en figure 12. Il entraîne un appui de l'organe expansible 24 et de la gaine interne 3 sur la paroi interne de l'enveloppe externe 2 apte à déployer le nez 14 par écartement des différents segments 15a, 15b, 15c, 15d. Cette situation est représentée en figure 14 où on remarque l'expansion de l'organe expansible 24 une fois libéré.

A ce niveau, l'autre organe expansible 25 est encore maintenu à l'intérieur de la gaine interne 3.

Pour une application à l'occlusion vasculaire, il est alors possible de réaliser une rotation de la gaine interne 3 de façon à modifier la position angulaire relative des organes expansibles 24, 25. A cet effet, le praticien utilise la poignée 7 pour en modifier la position angulaire. Bien entendu, dans cette application, il est nécessaire que la gaine interne 3 ait une possibilité de mouvement en rotation suivant l'axe longitudinal du dispositif. En outre, il est avantageux de prévoir des moyens de réglage de la position angulaire de la gaine interne 3, par exemple sous forme d'un repère 19 situé sur la poignée 7 en regard d'une pluralité de graduations 20 formées sur la surface extérieure du pousseur 4 à un endroit visible pour l'utilisateur. Il peut ainsi régler la torsion imposée à la partie intermédiaire 26 afin de régler le degré d'occlusion, ce qui peut également produire un réglage de la longueur de l'implant 10 en opérant un nombre de tours plus ou moins grand. Cette possibilité est illustrée par la double flèche de la figure 16.

Par enlèvement de l'entretoise 9, on procède au retrait de l'ensemble constitué par l'enveloppe externe 2 et la gaine interne 3 par un mouvement de translation vers l'arrière exercé au niveau de la poignée 7 pour la rapprocher de la poignée 12 jusqu'à la butée.

A ce stade, l'appui exercé par le pousseur 4 sur l'extrémité arrière de l'organe expansible 25 produit la libération dudit organe expansible 25 de la paroi interne de la gaine interne 3. Cette libération entraîne l'expansion de l'organe 25 pour sa mise en place sur la paroi vasculaire. On parvient ainsi à la situation illustrée en détail à la figure 18 où le nez 14 est reculé par rapport à la gaine interne 3, ce dernier étant lui-même reculé par rapport au pousseur 4. Bien entendu, ici, les positions de retrait relatif sont accentuées pour la bonne compréhension.

Le câble de guidage 11 peut être retiré ou a pu l'être précédemment. On notera que le nez 14 comporte un passage central résiduel 14 visible en vue de face à la figure 4 pour permettre l'introduction et le retrait du câble 11.

Les entretoises 8 et 9 sont ici constituées par des éléments présentant une section annulaire en croissant de lune de plus de 180°. L'ouverture restante permet de jouer sur l'élasticité de la matière de l'entretoise pour réaliser son enlèvement. Un exemple de forme des entretoises 8, 9 est présenté dans le cas de l'entretoise 8 aux figures 7 et 8. Bien entendu, une autre configuration est possible avec d'autres moyens permettant l'enlèvement de l'entretoise.

Les constituants principaux de l'invention sont, à titre d'exemple, en polyuréthane ou en polyéthylène.

### REFERENCES

1. Dispositif de dilatation d'un vaisseau
2. Enveloppe
3. Gaine interne
4. Pousseur
6. Poignée de l'enveloppe
7. Poignée de la gaine
8. Entretoise
9. Entretoise
10. Implant
11. Câble de guidage
12. Poignée du pousseur
14. Nez
15a, 15b, 15c, 15d. Segments
16a, 16b, 16c, 16d. Fentes
17. Jonction ponctuelle
18. Passage résiduel
19. Repère
20. Graduation
21. Dispositif de mise en place
22. Valve
23. Raccord
24. Organe expansible
25. Organe expansible
26. Partie intermédiaire
27. Chenal central

## Revendications

1. Dispositif (21) pour la mise en place d'un implant vasculaire (10) comprenant :
- un dispositif de dilatation (1) d'un vaisseau avec une enveloppe externe (2) et une partie d'extrémité effilée pour l'introduction dans le vaisseau, ladite partie d'extrémité étant constituée d'un nez (14) formé à l'extrémité distale de l'enveloppe externe (2) et le dispositif de dilatation (1) comportant des moyens d'ouverture du nez (14) avec au moins deux fentes longitudinales (16a, 16b, 16c, 16d) partageant le nez (14) en plusieurs segments (15a, 15b, 15c, 15d) déployables pour ouvrir le nez (14) ;
- un implant (10) placé dans l'enveloppe externe (2),
**caractérisé par le fait que**
- l'implant (10) comprend un premier organe expansible (24) s'appliquant sur la paroi interne de l'enveloppe externe (2), un deuxième organe expansible (25) creux et une partie intermédiaire (26) creuse et déformable en torsion ;
- il présente des moyens de translation de l'implant (10) relativement à l'enveloppe externe (2) de sorte à faire appuyer l'organe expansible (24) sur la paroi interne du nez (14) pour en déployer les segments (15a, 15b, 15c, 15d), les dits moyens de translation comportant une gaine interne (3) montée coulissante dans l'enveloppe externe (2) et poussant l'organe expansible (24),
- que le deuxième organe expansible (25) s'applique sur la paroi interne de la gaine interne (3) ;
- que la gaine interne (3) est montée coulissante et en rotation dans l'enveloppe externe (2).

2. Dispositif (1) selon la revendication précédente **caractérisé par le fait**
**qu'** il comporte une poignée de préhension (6) solidaire de l'enveloppe externe (2).

3. Dispositif (1) selon l'une quelconque des revendications précédentes **caractérisé par le fait**
**qu'**il comporte une poignée de préhension (7) solidaire de la gaine interne (3).

4. Dispositif (1) selon la revendication 3 en combinaison avec la revendication 2 **caractérisé par le fait**
**que** la poignée de préhension (7) de la gaine interne (3) est située en arrière de la poignée de préhension (6) de l'enveloppe externe (2) et qu'elle comporte une entretoise (8) amovible intercalée entres lesdites poignées (6,7) pour maintenir leur écartement.

5. Dispositif (1) selon l'une quelconque des revendications précédentes **caractérisé par le fait**
**que** les segments déployables (15a, 15b, 15c, 15d) sont joints ponctuellement le long des fentes (16a, 16b, 16c, 16d) en position de fermeture du nez (14).

6. Dispositif (1) selon la revendication précédente **caractérisé par le fait**
**qu'**il comporte une jonction ponctuelle (17) par fente (16a, 16b, 16c, 16d) entre les segments (15a, 15b, 15c, 15d).

7. Dispositif (1) selon l'une quelconque des revendications précédentes **caractérisé par le fait**
**que** le nez (14) comporte un passage central résiduel (18).

8. Dispositif (1) selon l'une quelconque des revendications précédentes **caractérisé par le fait**
**que** le nez (14) présente une mémoire de forme de sorte à être fermé par défaut lorsque les moyens d'ouverture sont inactifs.

9. Dispositif (1) selon l'une quelconque des revendications précédentes **caractérisé par le fait**
**qu'**il comporte un pousseur (4) monté coulissant dans la gaine interne (3) et apte à s'appliquer sur l'extrémité libre du deuxième organe expansible (25) ;

10. Dispositif (1) selon la revendication précédente **caractérisé par le fait**
**qu'**il comporte une poignée de préhension (12) solidaire du pousseur (4) située en arrière d'une poignée de préhension (7) solidaire de la gaine interne (3) et qu'il comporte une entretoise (9) amovible intercalée entre lesdites poignées (7, 12) pour maintenir leur écartement.

11. Dispositif (1) selon l'une quelconque des revendications précédentes **caractérisé par le fait**
**qu'**il comporte des moyens de réglage (19, 20) de la position angulaire de la gaine interne (3).

12. Dispositif (1) selon l'une quelconque des revendications précédentes **caractérisé par le fait**
**qu'**il comporte un chenal central (27) suivant l'axe de l'enveloppe externe (2) pour le passage d'un câble de guidage.

## Patentansprüche

1. Vorrichtung (21) zum Platzieren eines Gefäßimplantats (10) bestehend aus:
- einer Vorrichtung zum Aufweiten (1) eines Gefäßes mit einer äußeren Hülle (2) und einem sich verjüngenden Endstück zum Einführen in das Gefäß, wobei das Endstück aus einer Nase (14) besteht, die am distalen Ende der äußeren Hülle (2) ausgebildet ist und die Aufweitungsvorrichtung (1) Öffnungseinrichtungen für die Nase (14) mit mindestens zwei Längsspalten (16a, 16b, 16c, 16d) besitzt, welche die Nase (14) in mehrere Segmente (15a, 15b, 15c, 15d) unterteilen, die gespreizt werden können, um die Nase (14) zu öffnen,
- ein in der äußeren Hülle (2) platziertes Implantat (10),
**gekennzeichnet dadurch,**
- **dass** das Implantat (10) ein erstes expandierbares Organ (24) besitzt, dass sich an die Innenwand der äußeren Hülle (2) anlegt, weiterhin ein zweites, hohles, expandierbares Organ (25), sowie einen hohlen und in Torsion verformbaren Mittelteil (26);
- **dass** sie Einrichtungen zur translatorischen Bewegung des Implantats (10) gegenüber der äußeren Hülle (2) besitzt, so dass das expandierbare Organ (24) an die Innenwand der Nase (14) angedrückt wird, um die Segmente (15a, 15b, 15c, 15d) zu spreizen, wobei die Translationseinrichtungen ein verschiebbar in der äußeren Hülle (2) angeordnetes inneres Hüllrohr (3) besitzen, welches das expandierbare Organ (24) vorwärts stößt,
- **dass** das zweite, expandierbare Organ (25) sich an die Innenwand des inneren Hüllrohrs (3) anlegt;
- **dass** das innere Hüllrohr (3) verschiebbar und drehbar in der äußeren Hülle (2) angeordnet ist.

2. Vorrichtung (1) gemäß vorstehendem Patentanspruch, **gekennzeichnet dadurch, dass** sie einen Griff (6) besitzt, der mit der äußeren Hülle (2) fest verbunden ist.

3. Vorrichtung (1) gemäß einem der vorstehenden Patentansprüche, **gekennzeichnet dadurch, dass** sie einen Griff (7) besitzt, der mit dem inneren Hüllrohr (3) fest verbunden ist.

4. Vorrichtung (1) gemäß Patentanspruch 3 in Kombination mit dem Patentanspruch 2, **gekennzeichnet dadurch, dass** der Griff (7) des inneren Hüllrohrs (3) hinter dem Griff (6) der äußeren Hülle (2) angeordnet ist, und dass ein abnehmbares Zwischenstück (8) zwischen den genannten Griffen (6, 7) montiert ist, um ihren Abstand voneinander zu wahren.

5. Vorrichtung (1) gemäß einem der vorstehenden Patentansprüche, **gekennzeichnet dadurch, dass** die spreizbaren Elemente (15a, 15b, 15c, 15d) in geschlossener Stellung der Nase (14) punktweise entlang der Spalte (16a, 16b, 16c, 16d) aneinander anliegen.

6. Vorrichtung (1) gemäß vorstehendem Patentanspruch, **gekennzeichnet dadurch, dass** sie zwischen den Segmenten (15a, 15b, 15c, 15d) je Spalt (16a, 16b, 16c, 16d) eine punktweise Verbindung (17) besitzt.

7. Vorrichtung (1) gemäß einem der vorstehenden Patentansprüche, **gekennzeichnet dadurch, dass** die Nase (14) einen verbleibenden Durchgang (18) besitzt.

8. Vorrichtung (1) gemäß einem der vorstehenden Patentansprüche, **gekennzeichnet dadurch, dass** die Nase (14) ein Formgedächtnis besitzt, so dass sie, wenn die Öffnungsmittel inaktiv sind, normalerweise geschlossen ist.

9. Vorrichtung (1) gemäß einem der vorstehenden Patentansprüche, **gekennzeichnet dadurch, dass** sie einen Stößel (4) besitzt, der verschiebbar im inneren Hüllrohr (3) angeordnet ist und sich an das freie Ende des zweiten expandierbaren Organs (25) anlegen kann.

10. Vorrichtung (1) gemäß vorstehendem Patentanspruch, **gekennzeichnet dadurch, dass** sie einen Griff (12) besitzt, der mit dem Stößel (4) fest verbunden ist, der hinter dem Griff (7) liegt, welcher mit dem inneren Hüllrohr (3) fest verbunden ist und dass sie ein abnehmbares Zwischenstück (9) besitzt, das zwischen den Griffen (7, 12) angeordnet ist, um ihren Abstand voneinander zu wahren.

11. Vorrichtung (1) gemäß einem der vorstehenden Patentansprüche, **gekennzeichnet dadurch, dass** sie Einstellvorrichtungen (19, 20) für die Winkelstellung des inneren Hüllrohrs (3) besitzt.

12. Vorrichtung (1) gemäß einem der vorstehenden Patentansprüche, **gekennzeichnet dadurch, dass** sie einen zentralen, in Richtung der Achse der äußeren Hülle (2) verlaufenden Kanal (27) für einen Führungsdraht besitzt.

## Claims

1. Device (21) for the placing a vascular implant (10) comprising:
- a device (1) for dilating a vessel, with an outer casing (2) and a tapered end part for insertion into the vessel, said end part consisting of a nose (14) formed at the distal end of the outer casing (2) and the dilating device (1), with means (14) for opening the nose comprising at least two longitudinal slits (16a, 16b, 16c, 16d) dividing the nose (14) into several segments (15a, 15b, 15c, 15d) that can be deployed to open the nose (14);
- an implant (10) positioned in the outer casing (2),
**characterized in that**
- the implant (10) comprises a first expandable body (24) bearing on the inner wall of the outer casing (2), a second expandable hollow body (25) and a hollow intermediate part (26) that is torsionally deformable;
- it has means for ensuring the translatory movement of the implant (10) relative to the outer casing (2) so that the expandable body (24) bears on the inner wall of the nose (14) so as to deploy the segments (15a, 15b, 15c, 15d), the said translating means comprising an inner sheath (3) fitted to slide inside the outer casing (2) and to push the expandible body (24),
- the second expandable body (25) bears on the inner wall of the inner sheath (3);
- the inner sheath (3) is mounted to slide and rotate in the outer casing (2).

2. Device (1) according to the preceding claim **characterized in that** it comprises a gripping handle (6) forming an integral part with the outer casing (2).

3. Device (1) according to any preceding claim **characterized in that** it comprises a gripping handle (7) forming an integral part with the inner sheath (3).

4. Device (1) according to claim 3 in combination with claim 2 **characterized in that** the gripping handle (7) of the inner sheath (3) is located behind the gripping handle (6) of the outer casing (2) and comprises a removable spacer (8) positioned between the said handles (6,7) so as to maintain their spacing.

5. Device (1) according to any preceding claim **characterized in that** the deployable segments (15a, 15b, 15c, 15d) are joined at specific positions along the slits (16a, 16b, 16c, 16d) when the nose (14) is closed.

6. Device (1) according to the preceding claim **characterized in that** it comprises a joining (17) at specific positions by the slits (16a, 16b, 16c, 16d) between the segments (15a, 15b, 15c, 15d).

7. Device (1) according to any preceding claim **characterized in that** the nose (14) has a residual central passage (18).

8. Device (1) according to any preceding claim **characterized in that** the nose (14) has a shape memory so as to be closed by default when the opening means are not active.

9. Device (1) according to any preceding claim **characterized in that** it comprises a pusher (4) fitted to slide in the inner sheath (3) and adapted to bear on the free end of the second expandable body (25).

10. Device (1) according to the preceding claim **characterized in that** it comprises a gripping handle (12) forming an integral part with the pusher (4) located behind a gripping handle (7) forming an integral part with the inner sheath (3) and comprises a removable spacer (9) positioned between the said handles (7, 12) in order to maintain their spacing.

11. Device (1) according to any preceding claim **characterized in that** it comprises means (19, 20) for adjusting the angular position of the inner sheath (3).

12. Device (1) according to any preceding claim **characterized in that** it comprises a central channel (27) along the axis of the outer casing (2) for passage of a guidewire.
